# EUROPEAN PATENT SPECIFICATION

(11) **EP 2 259 814 B1**
(45) Date of publication and mention of the grant of the patent: **24.07.2013**
(21) Application number: 09715519.6
(22) Date of filing: 24.02.2009
(51) Int. Cl.: A61M 5/158, A61M 5/142

(54) **BUBBLE SHAPED MEMBRANE AND USE OF SUCH MEMBRANE IN A DEVICE**
BLASENFÖRMIGE MEMBRAN UND VERWENDUNG DIESER MEMBRAN IN EINER VORRICHTUNBG
MEMBRANE EN FORME DE BULLE ET UTILISATION DE CETTE MEMBRANE DANS UN DISPOSITIF

(30) Priority: 25.02.2008 DK 200800262; 25.02.2008 US 31227 P
(43) Date of publication of application: 15.12.2010
(73) Proprietor: Unomedical A/S, 3460 Birkerød (DK)
(72) Inventor: GYRN, Steffen, DK-4100 Ringsted (DK); HASTED, Søren, Bo, DK-4180 Sorø (DK)
(74) Representative: Zacco Denmark A/S
(86) International application number: PCT/EP2009/052149
(87) International publication number: WO 2009/106517

(56) References cited:
- WO-A1-98/26835
- WO-A1-2005/002649
- WO-A1-2007/071255

## Description

### The technical field

The invention relates to a device comprising soft membrane which is used when it is necessary to join units while keeping fluid paths of the units under sterile conditions. Such membranes are often used when medication or nutrients are transferred sub- or transcutaneously to a patient. Especially when a continuously flow of medication e.g. insulin or nutrients are supplied to a patient it is necessary to keep access of micro organisms under control.

### Prior art

WO 2007/071255 (fig. 19) describes the use of a soft membrane of similar type as the invention. Fig. 19 in this patent application shows an embodiment of a fluid tight connection between a reservoir and an injection part. This device comprises a delivery part where of only a reservoir 4 is shown in fig.19. The device is constructed of a reservoir where the outlet is covered by a bubble shaped deformable membrane 26; this membrane prevents that micro organisms access the reservoir when the delivery part is not joined to the injection part 1. The membrane does not only cover the tip of the connector needle 6 but covers or surrounds a larger part of the connector needle 6. The inlet of the injection part 1 is also covered by a deformable bubble shaped membrane 27 and the connector needle 6 is here fastened to the injection part 1 but the connector needle 6 could also be fastened to the delivery part 3, 4.

WO 2005/002649 (Fig. 3A, 3B) also refers to a device comprising a membrane according to the preamble of claim 1.

It is difficult to control the deformation of these known membranes and also the closed spaced inside the membrane might be wetted with the fluid which is being transferred to or from the device through the membrane.

### The invention

The object of the invention is to provide a device comprising a membrane completely covering an opening of a needle giving access to a space in the device. The membrane is made of an elastic material which can be penetrated by a needle, the membrane is fastened around the opening and the membrane protrudes from the opening and forms an air filled volume in front of the opening which air filled volume can be reduced in size when a pressure is put on the membrane from the outside, the inner surfaces of the membrane define a passage through which a needle can pass and at least a part of this passage is defined by having a reduced cross-section of air-filled volume. The reduced cross-section of the air-filled volume provides this part of the membrane with a certain rigidity which makes it maintain the shape it is provided with before being subjected to an external pressure. That the material is elastic means that it can be deformed when influenced by external forces and after the forces which deformed the material have been removed the material will return to the original shape. That the membrane is fastened around the opening does not mean it necessarily has a shape similar or corresponding to the opening although this might be the case, it only means that the contact between the membrane and the device provides a fluid tight closure for the whole opening. The "air filled volume" can be filled with any form of gas. Normally it will be gas used for sterilization mixed with air, and normally the volume will be filled at least mainly with air.

More specifically the invention relates to a device comprising a membrane according to claim 1.

The membrane can be made e.g. of silicone or a flexible thermoplastic material.

According to one embodiment the membrane is supported with walls of a rigid material on the inner surfaces e.g. in the form of a penetrating needle.

That the membrane is in a use position means that the membrane is mounted on or with the device with which it is supposed to be used e.g. as suggested in the present text the membrane is mounted in an infusion device where it can protect an inlet for medication.

According to one embodiment the second end of the membrane has an internal opening fitting around the outer contour of a protruding part on the holding part and that the membrane is fastened to the holding part by interference fit.

According to an embodiment of the membrane the cross-section of the air-filled volume in the passage is smaller than 2·[max cross-section of the opening from the device to the membrane].

The passage is formed by increasing the thickness of the membrane compared to parts of the membrane which does not form parts of the passage.

According to an embodiment of the membrane the space in the device is sterile. The device including the air filled volume is normally sterilized before use of the device and kept sterile during use of the device.

According to an embodiment of the membrane the properties of the material forming the membrane varies making it possible to predict how the air filled volume is reduced when subjected to an external pressure i.e. how the membrane is folded. The properties might e.g. be the thickness or the flexibility of the material.

The opening is provided at the end of a needle fastened unreleasably to the device providing a fluid path into the device and the needle is placed in the air filled volume and during periods where no pressure is put on the membrane the part of the needle extending into the air filled volume is completely surrounded by air. The needle will during periods where pressure is put on the membrane extend through the membrane and protrude from the outer surface of the membrane. Further the needle is a blunt needle or a pointy needle. A blunt needle is not able to cut through the membrane by itself but has to be presented with some kind of opening, a pointy needle can by itself cut through the membrane and it would not be necessary to provide the membrane with an opening.

According to an embodiment of the membrane the membrane can be penetrated by a blunt or a pointy needle.

A part of the membrane positioned closest to the device to which the membrane is fastened is thinner and more compliant and the material of the membrane positioned furthest away from the device is thicker and more rigid. "The part of the material positioned closest to the device" does not refer to a particular fraction of the material but more to a band of material extending along the full circumference of the material closest to the opening.

According to an embodiment of the membrane the material of the membrane positioned closest to the device has the thickness b₁ and has the form of a hollow truncated cone or a cylinder and the material positioned furthest away from the device has a thickness b₂ and has the form of a hollow cylinder which is closed at the end opposite the device, and b₂ > b₁.

According to an embodiment of the membrane a part of the membrane positioned between the material closest to the device having the thickness b₁ and the material positioned furthest away from the device having a thickness b₂, has a thickness varying from b₁ to b₂, according to one embodiment b₂ ≥ 1.5·b₁.

According to another arrangement of the membrane not in accordance with the present invention b₂ < b₁ and the distance between the rigid material supporting the inner surfaces of the membrane which could e.g. be a connector needle and the inner surfaces of the membrane is less than 0.5 mm.

A kit is also described comprising a device comprising a membrane according to any of the claims and a reservoir unit adapted to be joined to the device assuring a sterile transfer of fluid between the device and the joined reservoir unit.

The invention describes a device comprising a membrane according to any of the claims. The device may comprise a base part which comprises a surface plate and fastening means which fasten the base part to a subject, a fluid path is attached to the base part and the fluid path has at least to openings an inlet and an outlet for fluid, where one opening is protected by the membrane and the second opening is during use connected to a penetrating part which can transfer fluid to and from the subject in which it is inserted.

The invention will now be described with reference to the figures in which:
Figure 1 shows a cut-through view of a membrane according to the invention.
Figure 2A, B and C show an embodiment of a connection part including the internal parts.
Figure 2D and E show an enlargement of a cannula part which can be used together with the connection part.
Figures 3A and B show several arrangements not in accordance with the present invention.
Figure 3C shows several embodiments of sealings in the form of a bubble shaped membranes which can be used in connection with the invention.
Figure 4A shows an embodiment of a device where the delivery part is separated from the base part (seen from below).
Figure 4B shows an embodiment of a device where the delivery part is separated from the base part (seen from above).
Figure 4C shows the same embodiment as fig. 4A and 4B seen from another angle.
Figure 5 shows an enlargement of a second embodiment of a membrane according to the invention

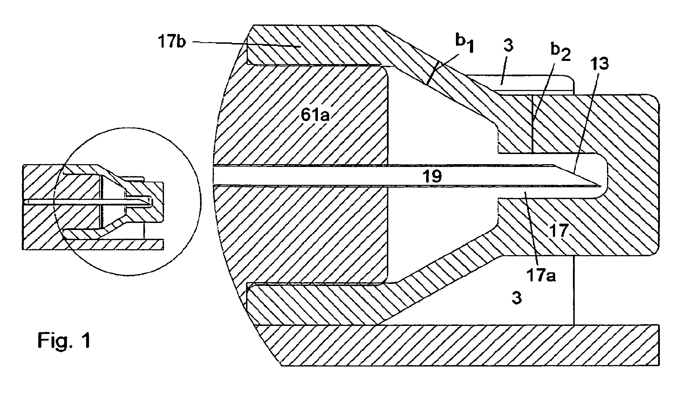

Fig. 1 shows an enlargement of a membrane 17 according to the invention. This bubble membrane 17 completely surrounds the part of the connector needle 19 which protrudes from the surface of the holding part 61 in which the connector needle 19 is embedded. The connector needle 19 does not touch the bubble membrane 17 when no pressure is put on the membrane 17 i.e. the connector needle 19 is completely surrounded by air which makes it possible to gas sterilize the connector needle 19; this is the state in which the membrane is shown in the figure. At the proximal end of the membrane 17 in a first area, the membrane 17 provides a passage 17a inside the membrane 17 having a reduced cross-section compared to a middle area of the membrane 17 surrounding a middle section of the needle 19. The passage 17a is defined by the inner surfaces of the walls of the membrane 17. The tip of the connector needle 19 is surrounded of quite thick walls constituted of the membrane forming a small air filled room around the tip of the connector needle 19 and providing the membrane material with a certain rigidity, while a middle part of the membrane closer to but free of the holding part has walls of approximately half this thickness providing flexibility of the walls, this has the result that when pressure is put on the membrane the thick walled part does not change shape, in stead the part of the membrane having reduced wall thickness i.e. the part closest to the holding part will give in, fold inward or outward, while the thick walled part is pressed toward the holding part 61. The membrane 17 further is provided with an open end 17b closest to the holding part 61; this open end 17b of the elastic membrane material fits around a protruding part 61a of the holding part 61. Due to friction between the inner surfaces of the open 17b end of the membrane 17 and the protruding part 61a, the membrane 17 stay put in the desired position i.e. the membrane is kept in position as a result of the interference fit.

Generally a membrane 17 according to the present invention surrounds a needle 19 and comprises three separate and identifiable areas which areas can be defined relative to the needle 19:
- a first area surrounds the tip of the needle 19, this area comprises relatively thick walls and small fluid volume around the needle 19,
- a second area surrounds a middle portion of the needle 19 having relatively thin walls and/or walls with folding features e.g. portions with very thin wall thickness or in another way prepared to ease length reduction of the membrane 17, and
- a third area which area is provided with means for or adapted to attach the membrane 17 to the holding parts 61 e.g. by fitting around a protruding part 61 a of the holding part 61.

Also a membrane 17 according to the present invention need not be abutted to or supported by surrounding walls of rigid material, this means that the membrane 17 is free to deform e.g. by bulking outwards in one or more bulk(s) depending on the folding features of the second area when pressure is put on the membrane 17. Without rigid material surrounding the sides of the membrane 17, also the sides of the membrane 17 - and not only the end - can also be penetrated by e.g. a syringe thereby adding fluid to the inside of the membrane 17 i.e. there is free access to at least part of the side area of the membrane 17.

The first area comprises the material of the membrane 17 which is positioned furthest away from the device i.e. the closed end piece which has to be penetrated by a needle 19 in order to form contact with e.g. a reservoir part placed outside the bubble shaped membrane 17. The membrane material in this first area is formed as a cavity having sides placed along the needle, a closed end to be penetrated by the needle 19, and an open end through which the needle 19 enters. The sides have a minimum material thickness b₂ which is large enough to make the first area non-deformable but the material thickness need not be constant all the way around the needle or in the whole length of the first area. The actual thickness or thickness distribution will depend on the choice of material and the dimensions e.g. length and diameter of the first area. Also the distance between the connector needle 19 and the membrane 17 will be of importance when determining how the membrane will be deformed when subjected to a pressure from the closed end i.e. if the distance between the outer surface of the connector needle 19 and the inner wall of the membrane is very small the walls of the membrane will when they are pushed back be guided by the contact with the connector needle 19 and not by the thickness i.e. the rigidity of the membrane material.

The second area comprises a portion of the membrane 17 which is positioned between the first and the third area. At least a part of the material of the membrane 17 in the second area has the thickness b₁, and the second area may have the form of one or more hollow truncated cone(s) or a cylinder(s) forming thick and thin parts in the membrane material in order to form folding features which define exactly where the membrane material will deform and how the material will deform. Normally the folding features are shaped to make the membrane material deform outward but as the rigid walls of the needle 19 assures that the fluid can keep flowing inside the needle 19, the membrane might as well deform inwards. Normally: b₂ > b₁ and for some materials often used for membranes of this type: b₂ ≥ 1.5·b₁. If the distance between the connector needle 19 and the membrane 17 in the first area is very small i.e. below 0.5 mm, the membrane thickness b2 might be smaller than b1, not in accordance with the present invention, as the membrane can then wrinkle or fold when being pushed back along the connector needle 19. In this case the connector needle 19 placed inside the membrane 17 functions as a guide and a support for the membrane 17.

The third area comprises a part of the membrane material which is shaped in such a way that it can be used to fasten the membrane 17 to the holding part 61. The third area can comprise membrane material in a thickness and flexibility which e.g. makes it adequate for fitting over and squeezing around a protruding part 61 a of the holding part 61 provided e.g. around the needle 19, or it can e.g. be shaped with an outward brim of material which makes it possible to squeeze the brim between two parts of the holding part 61.

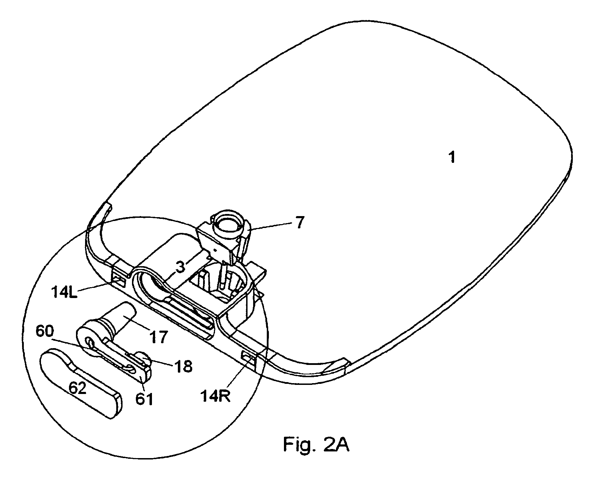

Fig. 2A-C show a membrane according to the invention used in a connection part 3 providing a fluid path to or from a reservoir to a patient. The connection part 3 is attached to a surface plate 1 which surface plate 1 is attached to a contact surface 2. The surface plate 1 is in this embodiment constructed of a molded plastic material and the contact surface is the proximal side of a mounting pad 2 which mounting pad 2 is unreleasably fastened to the surface plate 1 during manufacturing of the device.

The connection part 3 is attached to or integrated with the surface plate 1. According to the present embodiment the surface plate 1 and at least an outer cover of the connection part 3 is simply molded in one piece during manufacturing of the device. The connection part 3 forms a fluid path between e.g. a reservoir of medication, nutrients or the like to be supplied to a patient or a reservoir for liquid collected from a patient e.g. via a cannula part 7. Therefore the connection part 3 is provided with at least two openings, one opening at each end of the fluid path where the first opening 13 is an inlet or outlet opening receiving or delivering fluid to a reservoir 6 and the second opening 12 is an inlet or outlet opening receiving or delivering fluid to a cannula part 7. The connection part 3 might be provided with extra openings e.g. for injection of a second medication or nutrient or for letting the fluid in the fluid path get in contact with a sensor. Fig. 1 shows the reservoir 6 attached to the connection part 3 at the first opening 13 of the connection part 3. In the following the first opening 13 will be referred to as "inlet" and the second opening 12 will be referred to as "outlet" although the direction of the flow through the fluid path is not significant for the invention.

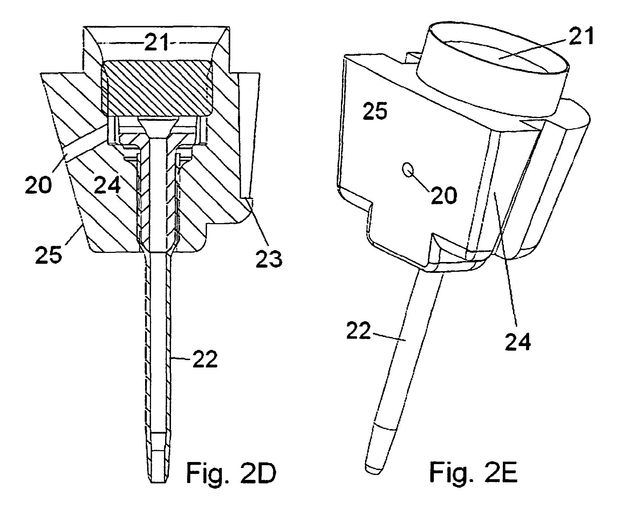

The connection part 3 is further provided with a cannula opening 12A which accurately fits around a cannula part 7 when the cannula part 7 is mounted in the connection part 3 i.e. the cannula opening 12A has the same shape or profile as the cannula part 7 and is just big enough to let the cannula part 7 pass through and then fit into the opening. In fig. 2A the cannula part 7 is shown in a position where the cannula part 7 is not fully inserted, normally the cannula part 7 would at this stage of insertion still be placed inside an inserter and it would not be visible. When the cannula part 7 is fully inserted, the upper surface i.e. the distal surface of the cannula part 7 is normally at level with or at a lower level than the outer surface of the connection part 3 around the cannula opening 12A. An enlargement of a cannula part 7 which can be used with the connection part 3 is shown in figs. 2D and 2E.

When the cannula part 7 has been fully inserted into the connection part 3, an opening 20 in a flat side surface 25 of the body 24 of the cannula part 7 corresponds to the opening 12 of the fluid path of the connection part 3 and fluid can flow from one part to the other. The opening 20 in the body 24 of the cannula part 7 might in the following be referred to as an "inlet" although the direction of the flow is not significant to the invention. The cannula part 7 is provided with a top opening 21 which allows access to the cannula part 7 by the use of a needle and with attachment means 23 in the form of an upward surface which fits under a not shown flexible part of the base part, and when the cannula part 7 is correctly positioned in the base part, it will be locked to the position and it will not be possible to remove the cannula part 7 again.

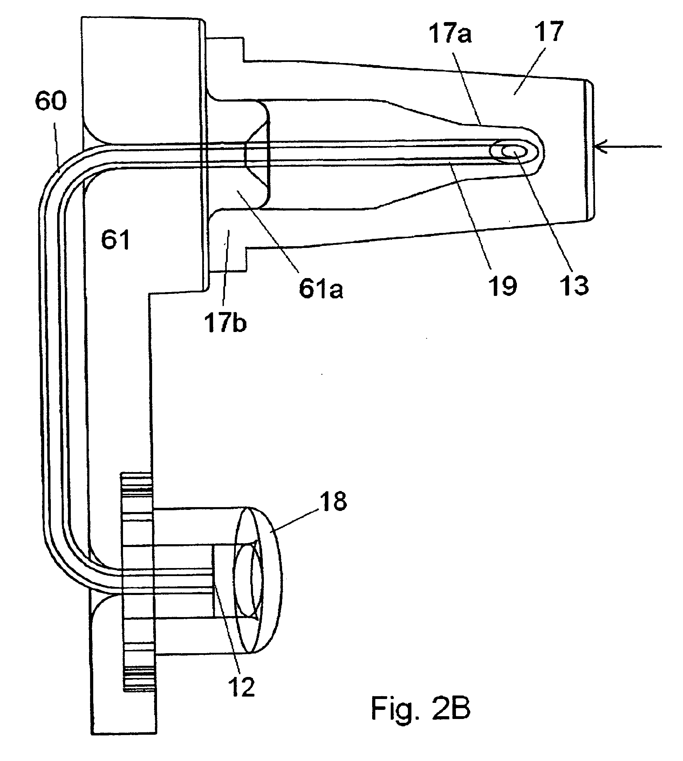

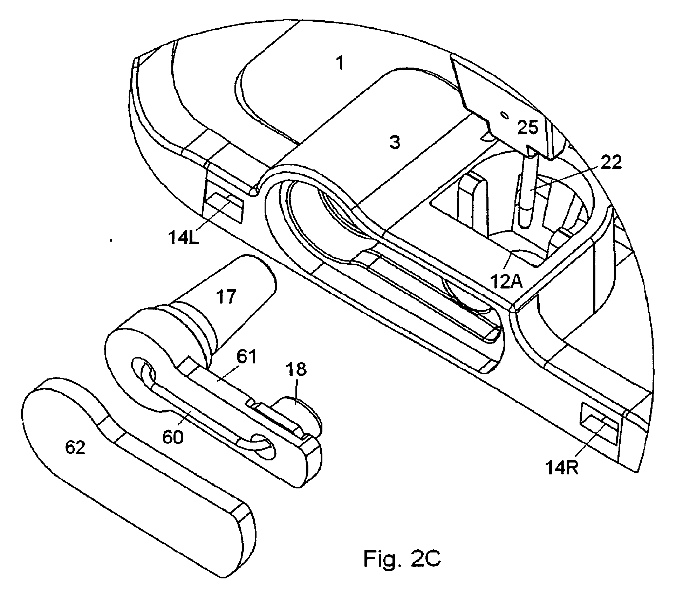

Fig. 2A show the connection part 3 in an exploded view where the internal holding parts 61 for a tube 60 providing a fluid path is shown. Fig. 2B shows a cut through the internal holding part 61 according to which it is possible to see the position of the tube 60. Fig. 2C shows an enlargement of the encircled part of fig. 2A.

According to the present embodiment the connection part 3 and the surface plate 1 is molded in one piece of a plastic material, the connection part 3 is provided with several openings, one opening 12A is prepared for fitting in the cannula part 7 and another opening is prepared for fitting in the internal parts of the connection part 3. The internal parts of the connection part 3 according to this embodiment comprises one tube which at two positions are bend in 90° i.e. both the inlet and the outlet end of the tube 60 points in the same direction perpendicular to the connecting part of the tube 60 where the connecting part of the tube 60 forms the fluid path between the two bending parts.

At one end the tube 60 is protected by the bubble shaped membrane 17 and at the other end the tube 60 is open and unprotected. The open tube end is surrounded by a sealing 18 which sealing is attached unreleasably to the holding part 61. When the internal parts has been placed in the corresponding opening in the connection part 3 a cover 62 accurately fitting in the opening is placed in level with the surface of the connection part 3 in such a way that the user experience a smooth surface which cannot be tampered with.

Fig. 2B shows an enlargement of the internal parts of the connection part 3. The holding parts 61 comprise a single molded part which is providing a stable embedment of the tube 60. The open end of the tube 60 opens into a volume surrounded by the sealing 18. The closed end of the tube 60 opens into a volume which volume is completely surrounded by an elastic membrane 17. "Completely surrounded" means that there is no free access to the surroundings, "elastic membrane" means that the membrane can be deformed and return to the original form and that the membrane can be penetrated by a needle, especially the connector needle 19 which is provided by the end of the tube 60 and is adapted to penetrate the membrane 17. The membrane 17 is fastened to the holding part 61 as the elastic material at the open end 17b of the membrane 17 squeezes around the protruding part 61 a of the holding part 61. The end of the tube 60 which constitutes the connector needle 19 is in this embodiment not in touch with the surrounding membrane 17 when the membrane is not subjected to a pressure from the outside. The connector needle 19 is surrounded by air, and the internal space surrounding the connector needle 19 has a several zones of cylindrical or conical shape i.e. a circular cross-section. The first zone closest to the holding part has approximately both inner and outer cylindrical having walls of approximately constant thickness. The second or middle zone has inner and outer walls formed as truncated cones and the variations of the inner and outer walls result in that the walls have a decreasing thickness towards the holding part 61. The third zone which can also be referred to as a passage 17a is closest to and surrounding the tip of the connector needle 19, both the inner walls and the outer walls of this zone are slightly cone-shaped but could as well be cylindrical or have an angular cross-section, the walls in this zone is thick although having slightly decreasing thickness towards the middle zone, the end of the zone is closed with a flat layer of membrane. When the a pressure is put on the flat end layer, the walls of the first zone of the membrane 17 will deform by bending inwards or outwards when the length of the membrane 17 is reduced as a result of the applied pressure. As the membrane 17 is placed behind the opening 13 when liquid is transferred e.g. from a reservoir to the connector needle 19, fluid will not be present in the volume surrounding the connector needle 19. When the pressure is removed and the membrane 17 returns to the position in front of the opening 13, fluid will normally not run out of the connector needle 19 as the connector needle 19 has a relatively small diameter (< 1 mm) and there is no free access of air at the cannula end of the tube 60.

Fig. 2C shows an enlargement of the enclosed field marked in fig. 2A.

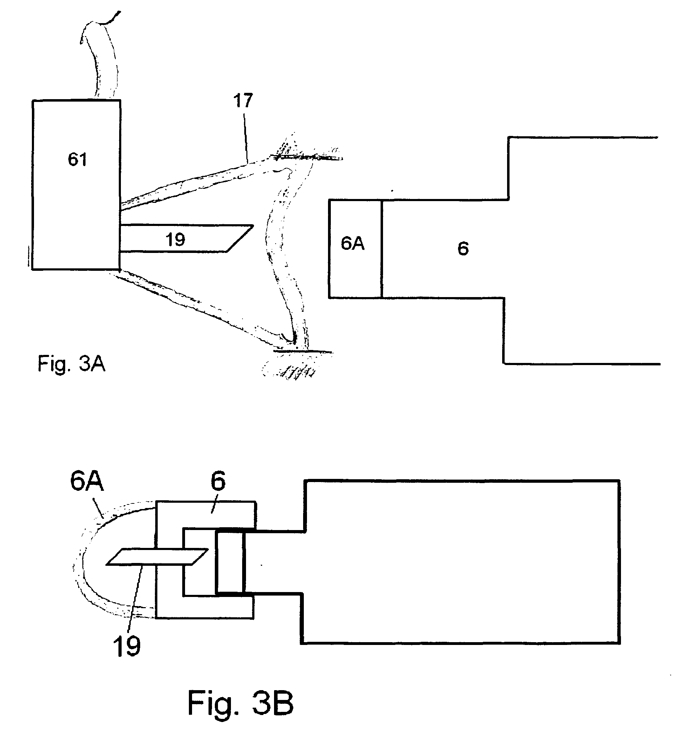

Fig. 3A shows an arrangement of a bubble shaped membrane not in accordance with the present invention. The reservoir 6, which is provided with an entrance protecting membrane 6A, is pushed toward the bubble membrane 17 covering the connector needle 19. The bubble membrane 17 is made of a flexible material which makes it possible for the membrane to be deformed to such an extent that the connector needle 19 can penetrate the protecting membrane 6A and extend into the reservoir thereby providing access to the fluid reservoir 6.

Fig. 3B shows an arrangement not in accordance with the present invention where a bubble membrane 6A is mounted at the outlet of a reservoir 6 which outlet can be connected to the fluid path of the connection path 3. The not shown end of the fluid path connecting to the reservoir 6 is provided with a membrane protecting the entrance of the fluid path during periods where the fluid path is not connected to the reservoir 6. According to this embodiment the fluid path need not be provided with a connector needle 19 as the connector needle 19 is part of the reservoir 6.

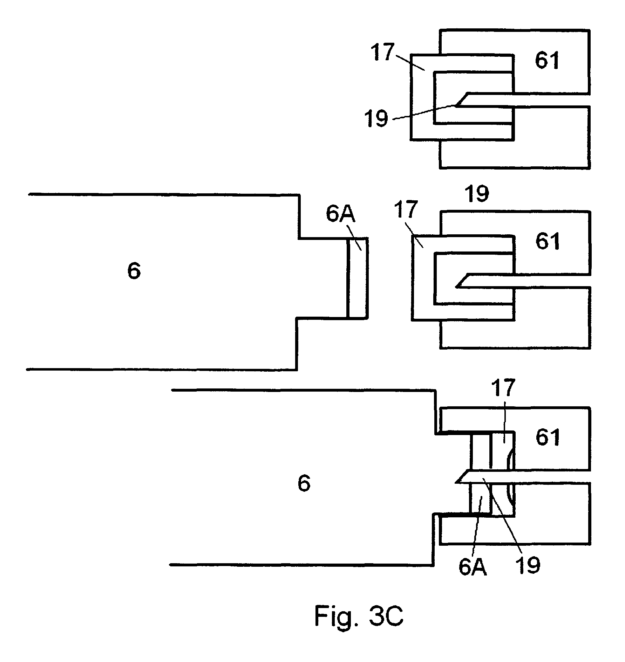

Fig. 3C shows an embodiment of a bubble membrane 17 and how the reservoir is pressed against the connector needle 19 in order to provide a fluid path for the medication contained in the reservoir 6. The bubble membrane 17 is flexible and is able to be reduced in size in such a way that it allows the entrance of the reservoir 6 to be pressed into the opening in the connection part 3 which surrounds the membrane 17 and the connector needle 19 i.e. the length of the membrane 17 can be reduced without the diameter of the membrane 17 being extended. According to the shown embodiment the material of the membrane will be folded inwards.

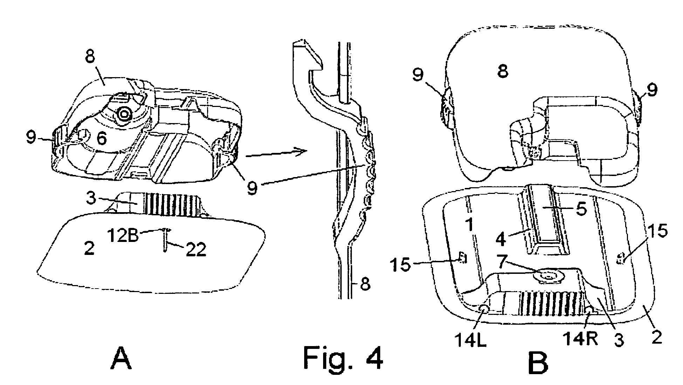

Figs. 4A-C show a device according to the invention comprising a base part comprising a fluid path provided with a membrane 17 and a delivery part comprising a reservoir 6, the two parts are in a position where they are separated from each other and they are shown from different angles. In fig. 4A the two parts are seen from below. This view shows an opening 12B through which the penetrating member 7 can be inserted through the base part and through which opening 12B the cannula 22 extends. From this view it is possible to see how the reservoir 6 can be positioned in the delivery part 8 and to see how two opposite positioned release handles 9 are placed at the edge of the delivery part 8. Further a longitudinal track corresponding to longitudinal raised guiding means 4 on the base part can be seen.

The two release handles 9 are formed as s-shaped bands where one end is fastened hinge-like to the housing of the delivery part 8 and the first curve in the s-shape is slightly extending the outer surface of the housing of the delivery part whereas the second curve is free i.e. not attached to the housing of the delivery part 8 and is provided with a hook-like shape which can fold around a part 15 protruding from the distal surface of the base part. When the delivery part is locked to the base part both release handles 9 are folded round a protruding part 15, when the delivery part 8 is to be removed from the base part, the two opposite release handles 9 are pushed together whereby the hook-like parts of the release handles 9 are released from the protruding parts 15 of the base part, and the delivery part can be moved backwards i.e. in the direction away from the cannula part 7 and removed from the base part in this direction.

In fig. 4B the two parts are shown from above. This view shows how the delivery part 8 of this embodiment can be joined to the base part by pushing the delivery part 8 down toward the guiding means 4 which in this case is a longitudinal raised platform having e.g. a metal lining 5 fastened to the top surface. The delivery part 8 is provided with corresponding means e.g. comprising a track corresponding to the raised platform 4. The corresponding means of the delivery part 8 can slide along the metal lining 5 of the raised platform 4 of the base part in the longitudinal direction. When the delivery part 8 arrives at its working position, the two release handles 9 engage respectively with the two protruding parts 15 protruding from the upper surface of the surface plate 1. When the delivery part 8 is in its working position it is locked in any horizontal direction by the release handles 9. The locking mechanisms make it possible to fasten and release the delivery device from the base part as often as needed i.e. a single-use base part can be combined with a multi-use delivery part.

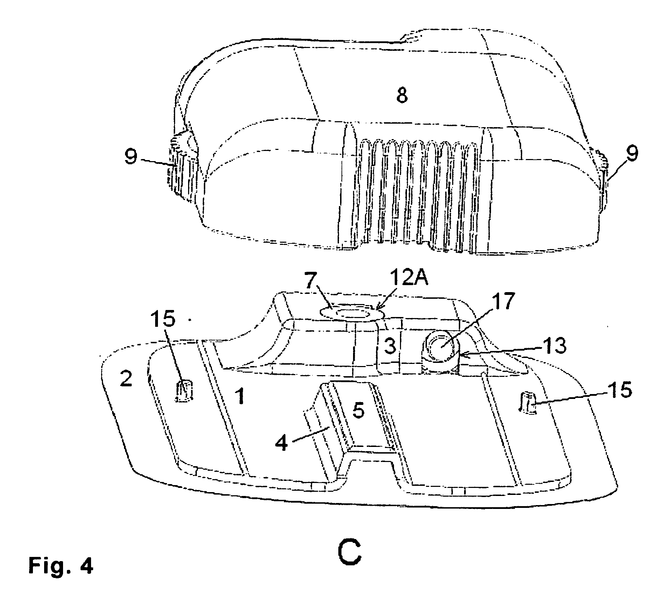

In fig. 4C the two parts are shown from the end opposite of where the inserter was fastened before insertion of the penetrating member. From this side it is possible to see the inlet opening 13 in the connection part 3 through which e.g. medication from the reservoir 6 can enter, the inlet opening 13 is protected with a membrane to prevent contamination with microorganisms. According to one embodiment the connection part 3 is provided with both a connector needle (not shown as it is placed behind the bubble shaped membrane) and a bubble shaped self closing membrane 17 and the reservoir 6 can be provided with a bubble shaped self closing membrane. Hereby a fluid path is established providing transfer of medication e.g. insulin or nutrients from the reservoir to the connector part 3. As both parts are provided with self closing membranes it will be possible to separate the two units from each other and rejoin them at a later time without the connection part 3 and thereby the patient being contaminated.

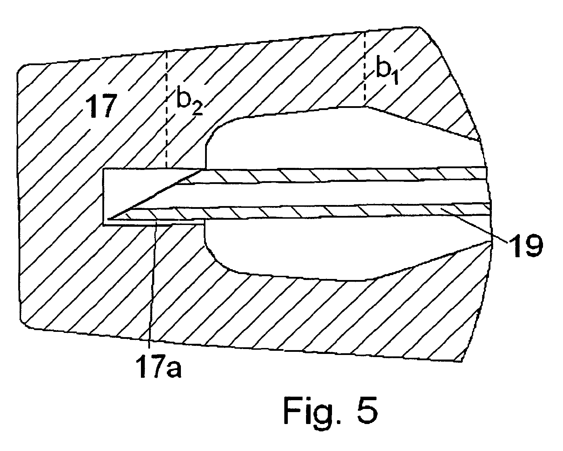

Fig. 5 shows an enlargement of a second embodiment of a membrane 17 according to the invention. This bubble membrane 17 also completely surrounds the part of the connector needle 19 which protrudes from the surface of the not shown holding part 61 in which the connector needle 19 is embedded. According to this embodiment the connector needle 19 is positioned so close to the membrane 17 that it might touch the bubble membrane 17 even when no pressure is put on the membrane 17 i.e. the passage 17a between the needle 19 and the membrane 17 represents a distance between membrane 17 and needle 19 close to 0, normally the distance will be less than 0.5 mm. When the membrane 17 is positioned this close to the connector needle 19, the needle 19 functions as guiding means when pressure is put on the membrane 17 which means that it will be easier to predict the folding of the membrane.

The needle 19 does not pierce the membrane 17 when no pressure is put on the membrane, this ensures filling of the membrane 17 with sterilizing gas such as Eto and distribution of the gas. The flexible membrane 17 should include a small vent e.g. a small cut in the side of the membrane 17 e.g. in the second area which cut opens when the membrane 17 is pushed over the needle 19. The vent allows for pressure relief.

## Claims

1. A device comprising a holding part (61) and a needle (19) with an opening (13), the device comprising a membrane (17) having a first end and a second end, the membrane completely covering the opening (13) giving access to a space in the device, wherein the membrane (17) is made of an elastic material penetrable by the needle (19), the membrane (17) is fastened around the opening (13) and the membrane (17) protrudes from the opening (13) and forms an air filled volume in front of the opening (13) which air filled volume can be reduced in size when a pressure is put on the membrane (17) from the outside, the inner surfaces of the membrane (17) defining a passage (17a) at the first closed end of the membrane (17) through which a needle (19) can pass, the membrane (17) being attached to the holding part (61) at the second end of the membrane (17), wherein the walls of the membrane (17) have a thickness and shape of the chosen membrane material making it possible, to maintain the protruding shape in a use position without the outer surfaces of the membrane (17) being supported with walls of rigid material **characterized in that** the membrane (17) has a constant or decreasing outer cross-section from where the membrane (17) is attached to the device and to the first end, and the passage (17a) is formed by increasing the thickness of the walls of the membrane (17) at the first end of the membrane (17) thereby reducing the inner air-filled cross-section of the membrane (17).

2. A device according to claim 1, wherein the membrane (17) surrounds the needle (19) and comprises
- a first area surrounding the tip of the needle (19), the first area comprising relatively thick walls and small fluid volume around the needle (19),
- a second area surrounding a middle portion of the needle (19), the second area having relatively thin walls and/or walls with folding features e.g. portions with very thin wall thickness or in another way prepared to ease length reduction of the membrane (17), and
- a third area provided with means for or adapted to attach the membrane (17) to the holding part (61) e.g. by fitting around a protruding part (61 a) of the holding part (61).

3. A device according to claim 1 or 2, wherein the second end of the membrane (17) has an internal opening (17b) fitting around the outer contour of a protruding part (61a) on the holding part (61) and that the membrane (17) is fastened to the holding part (61) by interference fit.

4. A device according to any of the preceding claims, wherein the membrane (17) is supported with walls of a rigid material on the inner surfaces of the membrane (17) e.g. in the form of a penetrating needle.

5. A device according to any of the preceding claims, wherein the cross-section of the air-filled volume in the passage (17a) is smaller than 2 · [max cross-section of the opening (13)].

6. A device according to any of the preceding claims, wherein the properties of the material forming the membrane (17) forming folding sections and making it possible to predict how the air filled volume is reduced when subjected to an external pressure.

7. A device according to any of the preceding claims, wherein the membrane (17) can be penetrated by a pointy needle.

8. A device according to any of the preceding claims, wherein the material of the membrane (17) positioned closest to the device has the thickness b₁ and has the form of a hollow truncated cone or a cylinder and the material positioned furthest away from the device has a thickness b₂ and has the form of a hollow cylinder which is closed at the end opposite the device, and b₂ > b₁.

9. A device according to claim 8, wherein a part of the membrane (17) positioned between the material closest to the device having the thickness b₁ and the material positioned furthest away from the device having a thickness b₂, has a thickness varying from b₁ to b₂.

10. A device according to claim 8 or 9, wherein b₂ ≥ 1.5·b₁.

11. A device according to any of the preceding claims, comprising
- a base part which base part comprise a surface plate (1), fastening means which fasten the base part to a subject, a fluid path (3) having at least two openings (12, 13): an inlet and an outlet for fluid, where one opening (13) is protected by the membrane (17) and the second opening during use connects to a penetrating part (7) transferring fluid to and/or from the subject in which it is inserted.

12. A device according to claim 11, wherein the opening (13) is provided at the end of a needle (19) which needle (19) is fastened unreleasably to the device and the needle (19) is placed in the air filled volume inside the membrane (17) and during periods where no pressure is put on the membrane (17) the part of the needle (19) extending into the air filled volume is completely surrounded by air.

13. A device according to claim 12, wherein the needle (19) during periods where pressure is put on the membrane (17) extends through the membrane (17) and protrudes from the outer surface of the membrane (17).

14. A kit comprising a device according to any of the claims 1-13 and a unit adapted to be joined to the device assuring a sterile transfer of fluid between the device and the joined unit.

## Patentansprüche

1. Vorrichtung umfassend ein Halteteil (61) und eine Nadel (19) mit einer Öffnung (13), welche Vorrichtung eine Membran (17) mit einem ersten Ende und einem zweiten Ende aufweist, welche Membran die Öffnung (13) völlig überdeckt, wodurch sich ein Zugang zu einem Raum in der Vorrichtung ergibt, wobei die Membran (17) aus einem elastischen Material hergestellt ist, welches mit der Nadel (19) durchstechbar ist, wobei die Membran (17) um die Öffnung (13) herum befestigt ist, und die Membran (17) von der Öffnung (13) vorspringt und vor der Öffnung (13) ein luftgefülltes Volumen bildet, welches luftgefüllte Volumen sich in der Größe reduzieren kann, wenn auf die Membran (17) ein Druck von außen ausgeübt wird, wobei die inneren Flächen der Membran (17) einen Durchgang (17a) an dem ersten geschlossenen Ende der Membran (17) definieren, durch welchen hindurch eine Nadel (19) passieren kann, wobei die Membran (17) an dem zweiten Ende der Membran (17) an dem Halteteil (61) befestigt ist, wobei die Wände der Membran (17) eine Dicke und eine Form aufweisen, die es ermöglichen, die vorspringende Form in einer Gebrauchsposition beizubehalten, ohne dass die Außenflächen der Membran (17) mit Wänden aus steifem Material unterstützt werden, **dadurch gekennzeichnet, dass** die Membran (17) einen konstanten oder sich vermindernden Querschnitt aufweist, von welchem die Membran (17) an der Vorrichtung und an dem ersten Ende befestigt ist, und der Durchgang (17a) durch Erhöhung der Dicke der Wände der Membran (17) an dem ersten Ende der Membran (17) gebildet ist, wodurch der innere, luftgefüllte Querschnitt der Membran (17) reduziert wird.

2. Vorrichtung nach Anspruch 1, wobei die Membran (17) die Nadel (19) umgibt und umfasst
- einen ersten Bereich, welcher die Spitze der Nadel (19) umgibt, wobei der erste Bereich relativ dicke Wände und ein kleines Flüssigkeitsvolumen um die Nadel (19) herum aufweist
- einen zweiten Bereich, welcher einen mittleren Abschnitt der Nadel (19) umgibt, wobei der zweite Bereich relativ dünne Wände und/oder Wände mit Faltmerkmalen, z.B. Abschnitte mit einer sehr dünnen Wanddicke, oder Abschnitte, die auf andere Art und Weise dazu vorbereitet sind, eine Längenreduktion der Membran (17) zu erleichtern, aufweist, und
- einen dritten Bereich, welcher mit Mitteln für Befestigung der Membran (17) am Halteteil (61) oder Mitteln, die für eine solche Befestigung ausgebildet sind, z.B. durch Montieren um einen vorspringenden Teil (61 a) des Halteteils (61) herum, versehen ist.

3. Vorrichtung nach Anspruch 1 oder 2, wobei das zweite Ende der Membran (17) eine innere Öffnung (17b) aufweist, welche um die Außenkontur eines vorspringenden Teils (61 a) an dem Halteteil (61) herum passt, und dass die Membran (17) durch eine Presspassung an dem Halteteil (61) befestigt ist.

4. Vorrichtung nach einem der vorgehenden Ansprüche, wobei die Membran (17) mit Wänden eines steifen Materials an den Innenflächen der Membran (17), z.B. in Form einer durchdringenden Nadel, unterstützt ist.

5. Vorrichtung nach einem der vorgehenden Ansprüche, wobei der Querschnitt des luftgefüllten Volumens in dem Durchgang (17a) kleiner als 2 · [maximaler Querschnitt der Öffnung (13)] ist.

6. Vorrichtung nach einem der vorgehenden Ansprüche, wobei die Eigenschaften des die Membran (17) bildenden Materials Faltabschnitte bilden und es ermöglichen, vorherzusagen, wie das luftgefüllte Volumen reduziert wird, wenn es einem Druck von außen ausgesetzt wird.

7. Vorrichtung nach einem der vorgehenden Ansprüche, wobei die Membran (17) von einer spitzen Nadel durchsetzt werden kann.

8. Vorrichtung nach einem der vorgehenden Ansprüche, wobei das Material der Membran (17), welches der Vorrichtung am nächsten angeordnet ist, die Dicke b₁ aufweist und die Form eines hohlen Kegelstumpfes oder eines Zylinders aufweist, und das Material, welches von der Vorrichtung am weitesten entfernt angeordnet ist, eine Dicke b₂ aufweist und die Form eines hohlen Zylinders aufweist, welcher an dem der Vorrichtung entgegengesetzten Ende geschlossen ist, und b₂ > b₁.

9. Vorrichtung nach Anspruch 8, wobei ein Teil der Membran (17), welcher zwischen dem der Vorrichtung am nächsten befindlichen Material mit der Dicke b₁ und dem von der Vorrichtung am weitesten entfernt befindlichen Material mit einer Dicke b₂, eine Dicke aufweist, die von b₁ bis b₂ variiert.

10. Vorrichtung nach Anspruch 8 oder 9, wobei b₂ ≥ 1.5·b1.

11. Vorrichtung nach einem der vorhergehenden Ansprüche, umfassend
- ein Basisteil, welches Basisteil eine Planscheibe (1), Befestigungsmittel, welche das Basisteil an einem Individuum befestigen, einen Fluidweg (3) mit mindestens zwei Öffnungen (12, 13), einen Einlass und einen Auslass für Flüssigkeit aufweist, wobei eine Öffnung (13) von der Membran (17) geschützt ist, und die zweite Öffnung während des Gebrauchs mit einem Durchdringungsteil (7) verbunden ist, welches Flüssigkeit in das Individuum oder aus dem Individuum, in welchem es eingesetzt ist, überträgt.

12. Vorrichtung nach Anspruch 11, wobei die Öffnung (13) an dem Ende einer Nadel (19) vorgesehen ist, welche Nadel (19) an der Vorrichtung lösbar befestigt ist, und die Nadel (19) in dem luftgefüllten Volumen innerhalb der Membran (17) angeordnet ist, und während Perioden, wo auf die Membran (17) kein Druck ausgeübt wird, der Teil der sich in das luftgefüllte Volumen erstreckenden Nadel (19) von Luft völlig umgeben ist.

13. Vorrichtung nach Anspruch 12, wobei sich die Nadel (19) während Perioden, wo auf die Membran (17) ein Druck ausgeübt wird, durch die Membran (17) hindurch erstreckt und von der Außenfläche der Membran (17) vorspringt.

14. Kit umfassend eine Vorrichtung nach einem der Ansprüche 1-13 und eine Einheit, die zur Verbindung mit der Vorrichtung ausgebildet ist, wodurch eine sterile Übertragung von Flüssigkeit zwischen der Vorrichtung und der verbundenen Einheit gewährleistet wird.

## Revendications

1. Dispositif comprenant une partie de maintien (61) et une aiguille (19) avec une ouverture (13), le dispositif comprenant une membrane (17) ayant une première extrémité et une deuxième extrémité, la membrane recouvrant complètement l'ouverture (13) donnant accès à un espace dans le dispositif, la membrane (17) étant faite d'un matériau élastique pénétrable par l'aiguille (19), la membrane (17) étant fixée autour de l'ouverture (13) et la membrane (17) faisant saillie à partir de l'ouverture (13) et créant un volume rempli d'air en avant de l'ouverture (13), ledit volume rempli d'air pouvant être réduit en taille quand une pression est exercée sur la membrane (17) venant de l'extérieur, les surfaces intérieures de la membrane (17) définissant un passage (17a) à la première extrémité fermée de la membrane (17) à travers laquelle une aiguille (19) peut passer, la membrane (17) étant fixée à la partie de maintien (61) à la deuxième extrémité de la membrane (17), les parois de la membrane (17) présentant une épaisseur et une forme du matériau choisi pour la membrane permettant de maintenir la forme en saillie dans une position d'utilisation sans les surfaces extérieures de la membrane (17) étant supportées par des parois en matériau rigide, **caractérisé en ce que** la membrane (17) présente une section transversale extérieure constante ou décroissante à partir de laquelle la membrane (17) est fixée au dispositif et à la première extrémité, et le passage (17a) est formé par augmentation de l'épaisseur des parois de la membrane (17) à la première extrémité de la membrane (17), réduisant ainsi la section transversale intérieure remplie d'air de la membrane (17).

2. Dispositif selon la revendication 1, dans lequel la membrane (17) entoure l'aiguille (19) et comprend
- une première zone entourant la pointe de l'aiguille (19), la première zone comprenant des parois relativement épaisses et un petit volume de fluide autour de l'aiguille (19),
- une deuxième zone entourant une portion centrale de l'aiguille (19), la deuxième zone ayant des parois relativement minces et/ou des parois avec des fonctions de pliage, p.ex. des portions avec une épaisseur de paroi très mince ou préparées d'une autre manière à faciliter la réduction de longueur de la membrane (17), et
- une troisième zone pourvue de moyens pour ou adaptée pour fixer la membrane (17) à la portion de maintien (61) p.ex. par ajustage autour d'une partie saillante (61 a) de la partie de maintien.

3. Dispositif selon la revendication 1 ou 2, dans lequel la deuxième extrémité de la membrane (17) présente une ouverture intérieure (17b) s'ajustant autour du contour extérieur d'une partie saillante (61 a) sur la partie de maintien (61) et que la membrane (17) est fixée à la partie de maintien (61) par l'ajustement avec serrage.

4. Dispositif selon l'une quelconque des revendications précédentes, dans lequel la membrane (17) est supportée par des parois d'un matériau rigide sur les surfaces intérieures de la membrane (17) p. ex. dans la forme d'une aiguille pénétrante.

5. Dispositif selon l'une quelconque des revendications précédentes, dans lequel la section transversale du volume rempli d'air dans le passage (17a) est inférieure à 2 · [section transversale maximale de l'ouverture (13)].

6. Dispositif selon l'une quelconque des revendications précédentes, dans lequel les propriétés du matériau formant la membrane (17) formant des sections de pliage et permettant de prévoir la manière dont le volume rempli d'air est réduit lorsqu'il est soumis à une pression extérieure.

7. Dispositif selon l'une quelconque des revendications précédentes, dans lequel la membrane (17) peut être pénétrée par une aiguille pointue.

8. Dispositif selon l'une quelconque des revendications précédentes, dans lequel le matériau de la membrane (17) positionné le plus proche au dispositif présente l'épaisseur b₁ et présente la forme d'un tronc de cône creux ou d'un cylindre et le matériau disposé le plus éloigné du dispositif présente une épaisseur b₂ et présente la forme d'un cylindre creux qui est fermé à l'extrémité opposée du dispositif, et b₂ > b₁.

9. Dispositif selon la revendication 8, dans lequel une partie de la membrane (17) positionnée entre le matériau le plus proche du dispositif ayant l'épaisseur b₁ et le matériau placé le plus éloignée du dispositif ayant une épaisseur b₂, présente une épaisseur variant de b₁ à b₂.

10. Dispositif selon la revendication 8 ou 9, dans lequel b₂ ≥ 1.5·b₁.

11. Dispositif selon l'une quelconque des revendications précédentes, comprenant
- une partie de base, ladite partie de base comprenant une plaque de surface (1), des moyens de fixation qui fixent la partie de base à un objet, un trajet de fluide (3) comportant au moins deux ouvertures (12, 13): une entrée et une sortie pour du fluide, une ouverture (13) étant protégée par la membrane (17) et la deuxième ouverture lors de l'utilisation se raccordant à une partie de pénétration (7) transférant du fluide vers et/ou à partir de l'objet dans lequel elle est insérée.

12. Dispositif selon la revendication 11, dans lequel l'ouverture (13) est pourvue à l'extrémité d'une aiguille (19), ladite aiguille (19) étant fixée de manière non libérable au dispositif, et l'aiguille (19) est placée dans le volume rempli d'air à l'intérieur de la membrane (17) et pendant les périodes où aucune pression n'est exercée sur la membrane (17), la partie de l'aiguille (19) s'étendant dans le volume rempli d'air est entièrement entourée par de l'air.

13. Dispositif selon la revendication 12, dans lequel l'aiguille (19) pendant des périodes où la pression est placée sur la membrane (17) s'étend à travers la membrane (17) et fait saillie de la surface extérieure de la membrane (17).

14. Jeu comprenant un dispositif selon l'une quelconque des revendications 1-13 et une unité adaptée pour être jointe au dispositif assurant un transfert stérile de fluide entre le dispositif et l'unité jointe.
